# EUROPEAN PATENT APPLICATION

(11) **EP 3 888 532 A1**
(43) Date of publication of application: **06.10.2021**
(21) Application number: 20167740.8
(22) Date of filing: 02.04.2020
(51) Int. Cl.: A61B 5/00, A61B 5/06

(54) **SENSOR POSITIONING WITH REMOVABLE PLACEMENT TRACKER**

(71) Applicant: Koninklijke Philips N.V., 5656 AG Eindhoven (NL)
(72) Inventor: JOHNSON, Mark, 5656 AE Eindhoven (NL); VOGTMEIER, Gereon, 5656 AE Eindhoven (NL); SISODIA, Rajendra Singh, 5656 AE Eindhoven (NL)
(74) Representative: Philips Intellectual Property & Standards

(57) **Abstract**

In order to facilitate the placement and the positioning of a sensor arrangement on the patient's body, a sensor positioning system is proposed that comprises a removable placement tracking arrangement with one or more placement trackers. Each placement tracker is removably mountable to one or more sensors. Accordingly, the one or more sensors are not required to contain any placement tracking functionality, and as such will be simpler and less expensive than the combined sensor/position tracker device.

## Description

### FIELD OF THE INVENTION

The present invention relates to sensor placement and positioning, and in particular, to a sensor positioning system and a method for positioning and placement of a sensor arrangement on a patient's body, an examination arrangement, a computer program element, and a computer readable medium.

### BACKGROUND OF THE INVENTION

Sensors play a big role during imaging by providing vital physiological condition of patients as well as timings and position marking during scans. The sensors may be of different types such as Electrocardiography (ECG), respiratory rate (RR), Photoplethysmogram (PPG), saturation of peripheral oxygen (SPo2), or even patches (temperature, skin conductance, perspiration, position markers). Input from the sensors may be used to auto-trigger a scan or specific part of scan, such as cardiac cycle or reconstruction to compensate for motion. Further, ECG/RR may be used as input for scan gating. Sensor placement and the position of a sensor during a scan of an imaging modality is essential but a complex process. If such sensors are not placed properly or are positioned wrongly, the scan may have artefacts or even need to be repeated.

Sensor placement on a patient's body may be a subjective process. They are based on physical palpation or using anatomical markers. Some sensors, such as SPo2, may be used by anybody, whereas sensors, such as "ECG with multi-channel", may need to be placed by an experienced staff. Some sensors, such as ultrasonic sensors, not only need to be placed properly without air gaps, but also need to be positioned properly e.g. to isolate a vascular, tissue, anatomical region with right angle, depth etc.

The scan from big modality requires multiple types of sensors, also referred as sensor arrangement. Such a sensor arrangement, which may comprise a plurality of sensors and different types of sensors, makes it even more complex for placement and position to ensure that not only they are placed properly relative to themselves but also with respect to each other. For example, ECG leads should be placed properly with respect and relative to each other and other sensors, such as ultrasonic, should be placed in such a way that they do not introduce interferences.

### SUMMARY OF THE INVENTION

There may be a need to facilitate placement and positioning of a sensor arrangement on the patient's body.
The object of the present invention is solved by the subject-matter of the independent claims, wherein further embodiments are incorporated in the dependent claims. It should be noted that the following described aspects of the invention apply also for the sensor positioning system, the examination arrangement, the method, the computer program element, and the computer readable medium.

According to a first aspect of the present invention, there is provided a sensor positioning system for positioning and placement of a sensor arrangement on a patient's body. The sensor positioning system comprises a sensor arrangement comprising at least one sensor, a placement tracking arrangement comprising one or more placement trackers, each placement tracker being removably mountable to one or more sensors, and a processor. The placement tracking arrangement is configured to track and provide a feedback about a position and/or orientation of the at least one sensor relative to at least one reference point on the patient's body to determine an actual position and/or orientation of the at least one sensor on the patient's body. The processor is configured to compare the actual position and/or orientation of the at least one sensor with a desired position and/or orientation of the at least one sensor and to generate an output signal indicating a difference between the actual position and/or orientation and the desired position and/or orientation of the at least one sensor.

In other words, a sensor positioning system is proposed for positioning and placement of one or more sensors relative to at least one reference point, e.g. the sternum, on a patient's body with a removable placement tracking arrangement. The at least one reference point may comprise a single reference point, two reference points, a triangulation of three reference points, or even more reference points. The reference points may be anatomical markers, such as sternum or carotid artery of the patient, which may be visible in the actual image to help positioning the sensors. In case there are no obvious anatomical markers in the actual image, one or more position markers may be used to provide one or more visible reference points in the actual image to help positioning the sensors. In an example, a transmitter may be placed at the at least one reference point on the patient's body to communicate with the placement tracking arrangement, which is configured to is configured to track and provide a feedback about a position and/or orientation of the at least one sensor relative to the transmitter, i.e. the at least one reference point, on the patient's body. The transmitter thus forms the basis for the positioning of the one or more sensors. The positions of the one or more sensors can be determined based on a relative measurement in view of the at least one reference point. In another example, at least one reference sensor may be used for positioning at the at least one reference point. A placement tracker may be mounted to the at least one reference sensor, which may form the basis for the positioning of the other sensors, as the positions and/or orientations of the other sensors are determined based on a relative position and/or orientation measurement in view of the at least one reference sensor. The at least one reference sensor may be a passive sensor, which is configured to sense (e.g. absorb or reflect) the imaging radiation and thus position the at least one reference point.

The sensor arrangement may comprise one or more sensors. Examples of the one or more sensors may include, but are not limited to, ECG, RR, PPG, SPo2, and patches, such as temperature, skin conductance, position markers and perspiration. The sensor arrangement may be associated with a position profile, for position and placement of its sensors as per profile. This profile may be transmitted to or stored on the processor of the sensor positioning system. The position profile may be the desired placement grid for different sensor types over a curved surface, e.g. the patient's body, wherein the placement grid points may be three-dimensional (3D) coordinate points, with or without a specified orientation of the sensor. It should be noted that a position profile may be scan-dependent, modality-dependent, patient-dependent, or based on empirical values. The position profile may comprise at least one reference point, wherein the at least one reference point may be associated with e.g. a reference transmitter, a reference sensor, etc., and the desired position(s) for the sensor(s) is defined with respect to the at least one reference point. The reference transmitter or the reference sensor may be anchored using an anatomical marker on the patient's body or via an anatomical feature detection from the reference sensor.

The placement tracking arrangement comprises one or more placement trackers configured to track and provide a feedback about a position and/or orientation of the at least one sensor relative to at least one reference point on the patient's body to determine an actual position and/or orientation of the at least one sensor on the patient's body. Examples of the placement trackers may include, but are not limited to, an optical shape sensor and a body channel communication sensor. The feedback may be provided every second, every few seconds, or every 200-1000 milliseconds to aid placement of the one or more sensors.

Instead of incorporating the placement trackers into the sensors, each placement tracker of the placement tracking arrangement is removably mountable to the one or more sensors. Accordingly, the sensor arrangement may comprise a plurality of sensors without placement tracking ability. In positioning the sensor arrangement, the placement tracking arrangement may be temporally mounted to the sensors of the sensor arrangement to assist in positioning these sensors. Once these sensors are correctly positioned, the placement tracking arrangement may be removed, thus leaving these sensors at the correct positions and/or orientations. Compare to sensors with integrated placement tracking ability, the use of removable placement tracking arrangement may reduce the cost and size of the sensors, and also facilitate the radiology workflow in Magnetic Resonance Imaging (MRI), hybrid linear accelerator combined with a magnetic resonance imaging (MR LINAC), Positron emission tomography-magnetic resonance imaging (PET-MRI) and other MR-hybrid systems, and also in any other scanning modality, such as CT, PET, diagnostic X-Ray, etc.

There are various ways of removably mounting a placement tracker to one or more sensors.

A first option may be a spring-loaded clip, whereby one component is pushed into another, deforming the clip when pushed. The clip expands after being pushed over a barrier (for example into a slot) and is therefore held together. The force required to remove the component can be tuned by choosing the strength of the spring.

A second option may be a type of bayonet fitting, whereby the fixture is a combination of a push and a small twist (reversed to remove). Twist would preferably be very small to allow removal after sensor is on the body.

Another option may be some type of elasticated bands, e.g. attached to the top of the holder, whereby the elasticated bands are removed aside, the component added and the bands allowed to return to their original position.

Further options may include Velcro type fastenings, screw type fastenings, etc. For example, screw type fastenings may be used for just a single sensor - not for use with a frame device.

In an example, the placement tracking arrangement may comprise one or more detachable cartridges. Each detachable cartridge is removably attachable to a placement tracker. Some detachable cartridges may be structured differently to accommodate different sensor formats. The detachable cartridge may make it easier to mount sensors of different sizes to a single placement tracking device format.

In an example, the placement tracking arrangement may comprise a frame device, which has a default layout of the placement trackers for a particular procedure, e.g. particular scan procedure. Such a frame device may ensure that the sensors are initially placed at approximately the correct relative positions and may assist in the speed of using the placement tracking feature to find the final position more efficiently. Optionally, a series of frames with different sizes may be provided for various patients having different body sizes. Optionally, the placement trackers may be added to the frame at all relevant positions. Optionally, cartridges may be placed in the placement trackers to receive the required array of sensors. The frame may also preferably have a standard and/or flexible interconnect for the sensor array. A special version of the frame may be a flexible sheet with sensors. The sensors may be already connected in a flexible way that bending and individual positioning of the sensors on the body could be done when the placement tracker guides to the best position. These pre-configured flexible sheets with integrated cheap sensors may be used as patient specific disposable as the sensor setting might be connected to the patient's body for several hours and might not be reusable.

Thus, the actual position of the at least one senor may be determined based on the at least one reference point and the relative position of the at least one sensor relative to the at least one reference point. The tracking of the relative position and/or orientation of the at least one sensor in view of the at least one reference point may be continuously performed using the placement tracking arrangement.

The processor may compare the actual position and/or orientation of the at least one sensor with the desired position and/or orientation of the at least one sensor. Further, the processor may generate an output signal based on this comparison. This output signal may indicate the difference between the actual position and/or orientation and the desired position and/orientation. Thus, the output signal may be a feedback signal to the user of the sensor positioning system. Further, the placement tracker itself may provide feedback for each placement via a feedback system, the feedback system is preferably a multi-color fibre optic or an RF transceiver device operating at body channel communication frequencies of around 100MHz.

It should be noted that the processor might be an electrical circuit, a control unit or a calculation device, which also comprises a memory device to store data. Further, the processor may also contain a transceiver system to receive the position profile or the desired positions and/or orientations or connect to an external system, which may act as feedback system.

It should be noted that the at least one sensor might be autonomously positioned and placed with the aid of e.g. a robot system, where the tracking coordinates are dynamically evaluated with respect to the placement profile and the robot system is guided considering the current coordinate and placement profile to position the at least one sensor.

Therefore, the use of the sensor positioning system ensures an easy and reliable positioning and placement of the sensor arrangement on a patient's body. Further, the sensor arrangement may be positioned faster even by an untrained staff during the scan procedure to reduce time and improve the performance.

In an autonomous or self-guided scan procedure, e.g. to minimize the role of a support staff for patient preparation during the scan, it may be required to have a self-guided mechanism that not only allows diverse sensors to be placed but also actively guides them for placements and informs about change of placement. Further, personalization of sensor placement on the patient's body for a scan may be needed to avoid errors during preparation.

According to an embodiment of the present invention, the placement tracking arrangement comprises one or more modular devices, each modular device having a respective placement tracker. Each modular device is structured to accommodate at least one sensor.

With such a modular set-up, it is possible to combine the placement tracker(s) with any sensor or any set of sensors.

In an example, some placement trackers may be structured differently to accommodate sensors of different sizes.

In another example, this could be assisted by adding a cartridge feature to easily mount sensors of different sizes to a single placement tracking device format. Thus, if different sensors need to be positioned on the body, a plurality of identical placement tracking devices having different cartridges may be used to accommodate the different sensor formats. With the cartridge feature, it will be possible to re-use the same placement trackers many times and for many different procedures.

According to an embodiment of the present invention, each modular device comprises a detachable cartridge that is removably mountable to the placement tracker of the respective modular device. The detachable cartridge is structured to accommodate one or more sensors, which are removably mountable to the detachable cartridge.

As discussed above, some detachable cartridges may be structured differently to accommodate different sensor formats. Therefore, a plurality of identical placement tracking devices may be used many times and for different scanning procedures by simply replacing the detachable cartridges to accommodate different sensor formats, e.g. different sensor sizes and/or sensor shapes.

According to an embodiment of the present invention, the placement tracker in each modular device has a universal size.

According to an embodiment of the present invention, the sensor positioning system comprises a frame device removably mountable to the placement tracking arrangement. The frame device is configured to accommodate each placement tracker of the placement tracking arrangement in a predefined position and/or predefined orientation that defines a default layout of the sensor arrangement on the patient's body.

The frame device may be any template device that defines a default layout of the sensor arrangement on the patient's body for a particular procedure, e.g. a particular scan procedure. In an example, the frame device may be made of a rigid material, such as rigid plastic panel. In an example, the frame device may be made of a flexible material, such as flexible sheet.

Such a frame device may ensure that the sensors are initially placed at approximately the correct relative positions and may assist in the speed of using the placement tracking feature to find the final position more efficiently.

According to an embodiment of the present invention, at least one placement tracker on the frame device comprises a detachable cartridge for accommodating one or more sensors.

According to an embodiment of the present invention, the frame device comprises a standard or flexible interconnect for the sensor arrangement.

With the standard or flexible interconnect, the sensors may communicate among themselves and/or passively monitor their relative positions.

According to an embodiment of the present invention, the frame device is a flexible sheet. The sensor arrangement is an integrated part of the flexible sheet.

These pre-configured flexible sheets with integrated cheap sensors may be used as patient specific disposable, as the sensor setting may be connected to the patient's body for several hours and might not be reusable.

According to an embodiment of the present invention, the at least one reference point comprises an anatomical marker of the patient's body.

Examples of the anatomical marker may include, but are not limited to, the sternum or the carotid artery of the patient.

According to an embodiment of the present invention, the one or more placement trackers comprise at least one of an optical shape sensor and a radio frequency (RF) transceiver device operating at a body channel communication frequency.

One approach for positioning of sensors on the patient's body is the use of one or more optical shape sensors which are known to be configured to track and to provide a feedback about the position of a sensor inside a body, e.g. tip of a needle in a catheter using shape sensing but also function to provide feedback of position outside the body. For example, the placement tracker may be based on dual-layer fibre Bragg grating arrays with orthogonal mesh structure, which enables 3D bi-directional shape sensing. Furthermore, 3D shape sensing is proposed to detect the surface curvature, e.g. to detect strain. Alternatively or additionally, the proposed sensor positioning system may use a 3D shape sensing technique to provide a self-referencing position and placement guidance for the sensor arrangement. Further, measuring data and feedback information, such as colors and light patterns may be transmitted through the optical shape sensor. In particular, the shape of the optical shape sensor may be determined by the colour of photons in the fibre optics cable. Furthermore, since an optical link exists between the reference sensor and other sensors, they can also communicate over this optical link about the position and/or orientation and transmit measurement data to the processor for further processing.

A further approach for positioning of sensors on the patient's body is the body channel positioning approach. Hereby, each sensor may be provided with a pair of capacitive electrodes (e.g. two parallel electrodes), which capacitively contact the skin and can both transmit and receive signals propagated across and/or over the surface of the body. In some situations there are advantageously multiple capacitive electrode pairs on a given sensor e.g. to assist in positioning and orientation. Further, in this case the sensors may be equipped with a guiding system such as a LED or a speaker to provide feedback over the actual position and/or orientation of the at least one sensor to the user.

According to an embodiment of the present invention, the processor is configured to provide a guidance for the placement of the at least one sensor at the desired position and/or orientation based on the comparison of the actual position and/or orientation with the desired position and/or orientation of the at least one sensor.

In an example, the guidance for placement of the at least one sensor may be provided to a user, e.g. in form of an optical and/or acoustical feedback. For example, the optical feedback may be displayed on a display and the acoustical feedback may be emitted by a speaker. Thus, the user may be actively guided to place the at least one sensor at the desired position and/or orientation on the patient's body. For example, the sensor positioning system may display that the at least one sensor should be positioned and placed further right or left, and/or should be rotated for 15°.

For example, the optical feedback and/or the guidance for the placement of the at least one sensor comprises different colours and/or different light patterns. In other words, the guidance for the user may be performed by different colors or light patterns including spatial or temporal light pattern, i.e. animation. The guidance for the patient could be done via a simple color-coding, which is explained to the patient before. For example, colors as green, yellow, blue and purple could be used to indicate the movement directions (up, down, left, and right) for reaching the desired position and/or orientation - a red light could be used to stop movement and indicate "best position". If the optical fibres are used as placement tracker, the optical fiber itself could be used as optical interface to visualize the color. In case of the body channel network the sensor itself may be equipped with a multi-color LED as user interface.

Flashing lights might indicate a problem with the sensor position and/or orientation that might have moved or in case of skin contact sensors, the interface condition, e.g. gel, might have changed. Thus, the optical feedback can be used to indicate the need for action. This may also be performed in a synchronized way with an examination arrangement, as no position and/or orientation change should be done during image acquisition but only before the next scan. Audio guidance might support the patient actions.

The sequence of sensor placement can also be indicated by the optical interface either by a unique colour, e.g. white flashing indicating the sensor that has to be placed next, and/or temporal switching sequence.

In case of display availability in the preparation area and/or inside the examination apparatus when the sensors should be placed the synchronized visualization of sensor number and senor position and/or orientation on the body can be shown.

In another example, the patient may place the sensor arrangement on his body himself. Assuming the patient is lying down for an MRI or CT scan, a ceiling mounted mirror could allow the patient to see himself and the sensor arrangement from above. In this case, the examination arrangement may have built-in lighting elements to guide the user. For example, if the right hand edge lights up green it means that the user needs to move in the direction of that edge. If it lights up orange it means that the user is almost there, if it lights up red the user needs to stop. If the user overshoots and needs to reverse motion then the left hand edge lights up.

In a further example, the guidance for placement of the at least one sensor may be signified to a controller, which is configured to control and guide a robot system to autonomously place the at least one sensor at the desired position and/or orientation on the patient's body. The autonomous sensor positioning system may be beneficial for an autonomous or self-guided scan procedure, e.g. to minimize the role of support staff for patient preparation during the scan.

According to a second aspect of the present invention, there is provided an examination arrangement. The examination arrangement comprises an examination apparatus for examination of an object of interest and a sensor positioning system according to the first aspect of the present invention and any associated example.

The examination apparatus may be an MRI, CT, PET or DXR (Diagnostic X-Ray), or any other scanning apparatus. Further, the sensor positioning system and the examination apparatus may interact, such that the examining apparatus transmits the desired positions of the at least one sensor of the sensor arrangement to the sensor positioning system. Thus, an optimized measurement of vital data of the patient may be achieved. Further, the examination apparatus may trigger measurements of the sensor arrangement for or based on the measurement data of the sensor arrangement that the examination apparatus may be triggered. For example, the respiratory cycle may be determined based on the measurement data und based on the respiratory cycle the examination apparatus may be controlled. Further, the examination apparatus may be configured to obtain image data of the patient's body to determine the desired positions for the sensor arrangement.

According to a third aspect of the present invention, there is provided a method for positioning and placement of a sensor arrangement on a patient's body. The method comprises the steps of:
- mounting a sensor arrangement to a placement tracking arrangement, wherein the sensor arrangement comprises at least one sensor, and wherein the placement tracking arrangement comprises one or more placement trackers, each placement tracker being removably mountable to one or more sensors;
- tracking, by the placement tracking arrangement, and providing a feedback about a position and/or orientation of the at least one sensor relative to at least one reference point on the patient's body by the placement tracking arrangement to determine an actual position and/or orientation of the at least one sensor on the patient's body; and
- comparing, by a processor, the actual position and/or orientation with the desired position and/or orientation of the at least one sensor to generate an output signal indicating a difference between the actual position and/or orientation and the desired position and/or orientation of the at least one sensor.

In other words, at least one sensor may be firstly attached to the placement tracking arrangement, which may be a modular system to accommodate different sensors or a frame to accommodate multiple placement trackers and/or modular devices. Secondly, the at least one sensor is positioned relative to at least one reference point using the placement tracking arrangement. Finally, the placement tracking arrangement may be detached from the at least one sensor, leaving the at least one sensor at the correct position for the subsequent procedure, such as scan procedure.

According to a third aspect of the present invention, there is provided a computer program element for controlling a sensor positioning system according to the first aspect of the present invention and any associated example or an examination arrangement according to the second aspect of the present invention, which when being executed by a processor is configured to carry out the method according to the third aspect of the present invention.

According to a fourth aspect of the present invention, there is provided a computer readable medium having stored thereon the program element according to the third aspect of the present invention.

Advantageously, the benefits provided by any of the above aspects equally apply to all of the other aspects and vice versa.

In this detailed description, a person skilled in the art should note that quantitative qualifying terms such as "generally," "substantially," "mostly," and other terms are used, in general, to mean that the referred to object, characteristic, or quality constitutes a majority of the subject of the reference. The meaning of any of these terms is dependent upon the context within which it is used, and the meaning may be expressly modified.

The terms "removably attached", "removably attachable", "removably mounted", "removably mountable", and variations thereof may refer to two components connected or connectable such that the components remain connected when no separation force is applied to one or both of the components. This means that they can be separated without causing substantial permanent deformation or rupture or otherwise detrimentally affecting the potential for the components to be re-used. The required separation force generally exceeds the force generated during the wearing of the article.

The term "position and/or orientation" includes the position in coordinates and the orientation with respect to the coordinate system. As coordinate system, Cartesian coordinates may be used and the orientation may be determined based on the rotation around the coordinate axes.

The term "controller" is used generally to describe various apparatus relating to the operation of an apparatus, system, or method. A controller can be implemented in numerous ways (e.g., such as with dedicated hardware) to perform various functions discussed herein. A "processor" is one example of a controller, which employs one or more microprocessors that may be programmed using software (e.g., microcode) to perform various functions discussed herein. A controller may be implemented with or without employing a processor, and also may be implemented as a combination of dedicated hardware to perform some functions and a processor (e.g., one or more programmed microprocessors and associated circuitry) to perform other functions. Examples of controller components that may be employed in various embodiments of the present disclosure include, but are not limited to, conventional microprocessors, application specific integrated circuits (ASICs), and field-programmable gate arrays (FPGAs).

In various implementations, a processor or controller may be associated with one or more storage media (generically referred to herein as "memory," e.g., volatile and non-volatile computer memory). In some implementations, the storage media may be encoded with one or more programs that, when executed on one or more processors and/or controllers, perform at least some of the functions discussed herein. Various storage media may be fixed within a processor or controller or may be transportable, such that the one or more programs stored thereon can be loaded into a processor or controller so as to implement various aspects of the present disclosure discussed herein. The terms "program" or "computer program" are used herein in a generic sense to refer to any type of computer code (e.g., software or microcode) that can be employed to program one or more processors or controllers.

It should be appreciated that all combinations of the foregoing concepts and additional concepts discussed in greater detail below (provided such concepts are not mutually inconsistent) are contemplated as being part of the inventive subject matter disclosed herein. In particular, all combinations of claimed subject matter appearing at the end of this disclosure are contemplated as being part of the inventive subject matter disclosed herein.

These and other aspects of the invention will be apparent from and elucidated with reference to the embodiment(s) described hereinafter.

### BRIEF DESCRIPTION OF THE DRAWINGS

In the drawings, like reference characters generally refer to the same parts throughout the different views. Also, the drawings are not necessarily to scale, emphasis instead generally being placed upon illustrating the principles of the invention.
Fig. 1 schematically shows a sensor positioning system, in accordance with some embodiments of the present disclosure.
Fig. 2A schematically shows a placement tracking arrangement, in accordance with some embodiments of the present disclosure.
Fig. 2B schematically shows the placement tracking arrangement of Fig. 2A mounted to a sensor arrangement.
Fig. 2C schematically shows the placement tracking arrangement of Fig. 2A demounted from the sensor arrangement.
Fig. 3 schematically shows a placement tracking arrangement, in accordance with some further embodiments of the present disclosure.
Fig. 4 schematically shows a placement tracking arrangement, in accordance with some further embodiments of the present disclosure.
Fig. 5 schematically shows a patient with a sensor positioning system, in accordance with some embodiments of the present disclosure.
Fig. 6 schematically shows an examining table with a patient and the sensor positioning system, in accordance with some embodiments of the present disclosure.
Fig. 7 shows a flowchart of a method, in accordance with some embodiments of the present disclosure.

### DETAILED DESCRIPTION OF EMBODIMENTS

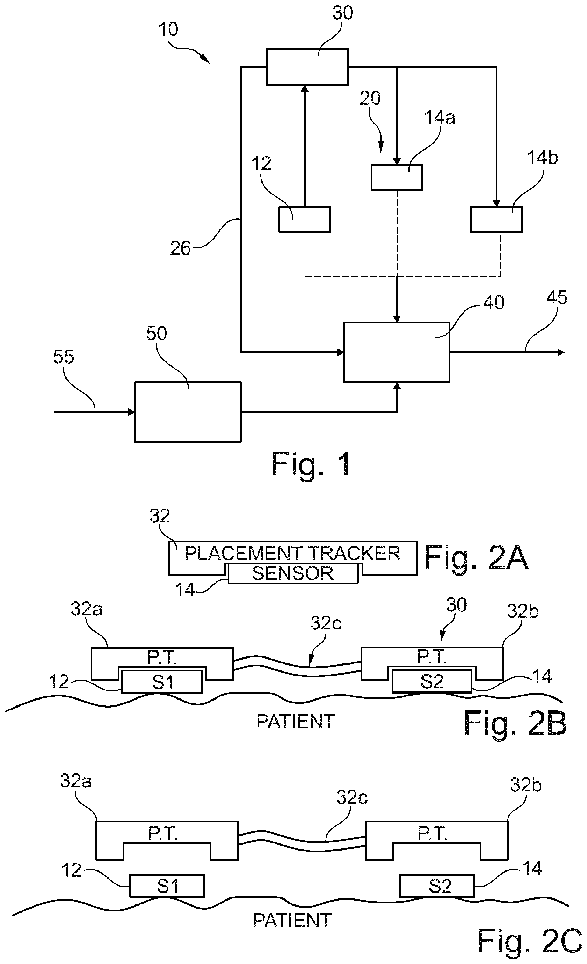

Fig. 1 schematically shows a sensor positioning system 10 according to some embodiments of the present disclosure. The sensor positioning system 10 comprises a sensor arrangement 20, a placement tracking arrangement 30, and a processor 40.

The sensor arrangement 20 comprises at least one sensor 14, such as sensor 14a and sensor 14b illustrated in Fig. 1. Although two sensors are illustrated in Fig. 1, it should be understood that also a single sensor or more than two sensors might be used. The sensors 14a and 14b of the sensor arrangement 20 are configured to be placed on a patient's body to measure vital data of the patient. Examples of the at least one sensor 14 may include, but are not limited to, an ECG sensor, a respiratory rate sensor, a PPG sensor, a SPo2 sensor, a temperature sensor, a skin conductance sensor, an ultrasonic sensor, a position marker, an Automated external defibrillator (AED) pad and/or perspiration sensor. The at least one sensor 14 is not required to contain any placement tracking functionality and as such will be simpler and less expensive than the combined sensor/position tracker device.

The placement tracking arrangement 30 comprises one or more placement trackers 32. Each placement tracker 32 is removably mountable to one or more sensors 14, e.g. by using a spring-loaded clip, bayonet fitting, elasticated bands, Velcro type fastenings, crew type fastenings, etc. The placement tracking arrangement 30 is configured to track and provide a feedback about a position and/or orientation of the at least one sensor 14 relative to at least one reference point 12 on the patient's body to determine an actual position and/or orientation of the at least one sensor 14 on the patient's body. In an example, the placement tracking arrangement 30 may comprise one or more optical shape sensors that are connected to the processor 40 and transmit the measurement data to the processor 40. The one or more optical shape sensor may guide visible light, different colors or different light patterns to provide an optical feedback to the user of the sensor position system 10. In another example, the placement tracking arrangement may comprise one or more radio frequency (RF) transceiver device operating at a body channel communication frequency, e.g. around 100MHz. For example, the sensors 14a, 14b of the sensor arrangement 20 may be provided with a pair of capacitive electrodes (e.g. 2 parallel electrodes), which capacitively contact the skin of the patient and can both transmit and receive signals propagated across the body.

The at least one reference point 12 may define a reference position and/or orientation. For example, the at least one reference point 12 may comprise an anatomical marker of the patient's body, such as the sternum or the carotid artery of the patient. The reference position and/or orientation may be determined based on an image, such as a MRI, CT or X-ray image, of the patient's body, based on a previous body scan of the patient or based on empirical values.

For example, the reference position and/or orientation defined by the at least one reference point may be determined based on a deterministic algorithm, which is based on position, weightage, computational capabilities, and/or sensing and power attributes. Thus, to determine the reference position and/or orientation of the at least one reference point, deterministic algorithms may be used to elect the master reference point(s) based on position/orientation, weightage, computational capabilities, sensing and power attributes. Weightage may be determined by various factors including the positioning data of earlier scans of same patient. The accuracy and stability of previous scans of the same/similar patient with respect to movement and quality of the scans influence the value of weightage. This addresses the need of personalization of sensor placement on the patient for scan to avoid errors during preparation. Further, multiple reference positions may be determined to deal with sensor node failures. After the election of reference positions, all sensors will automatically determine their relative positions.

A reference transmitter or a reference sensor may be used for positioning at the at least one reference point 12. In case of a reference sensor, the processor may be configured to determine whether the reference sensor is accurately placed at the at least one reference point on the patient's body by an iterative evaluation of the measurement quality of the reference sensor. Thus, the measurement data of the reference sensor may be analyzed by the processor in view of measurement quality. If the measurement quality exceeds a predefined threshold, the reference sensor may be considered to be accurately placed at the reference position and/or orientation on the patient's body.

The processor 40 is configured to compare the actual position and/or orientation of the at least one sensor with a desired position and/or orientation of the at least one sensor and to generate an output signal 45 indicating a difference between the actual position and/or orientation and the desired position and/or orientation of the at least one sensor 14. The desired position and/or orientation for the at least one sensor 14 may be stored in the sensor positioning system 10, e.g. 5 cm apart from the at least one reference point, or may be received by an optional receiver 50. The optional receiver 50 is configured to receive data 55, which may comprise e.g. desired positions for the at least one sensor 14 of the sensor arrangement 20, i.e. sensor positioning profile. In addition, the desired position and/or orientation for the at least one sensor 14 may be determined based on image data, on a previous body scan of the patient or on empirical values. The received position and/or orientation data 55 for the at least one sensor 14 of the sensor arrangement 20 may be received via wire or wireless, e.g. via Bluetooth or Wi-Fi. These position and/or orientation data 55 may be stored in the receiver 50 and be transmitted to the processor 40. Further, also the measurement data from the at least one sensor 14 of the sensor arrangement 20 may be transmitted to the processor (indicated by dotted lines) as well as the actual position and/or orientation 26 of the at least one sensor 14 tracked by the placement tracking arrangement 30.

In an example, the output signal 45 may be an optical feedback, such as a light, colors, or light patterns. For example, the processor may instruct a LED to emit a red light or a flashing light as long the actual position and/or orientation of the at least one sensor 14 is not equal to the desired position and/or orientation. If the actual position and/or orientation of the at least one sensor is equal to the desired/orientation position, the LED may emit a green light or a permanent light. The output signal 45 of the processor 40 may guide the user, e.g. a patient, of the sensor position system 10 for positioning the at least one sensor 14 at the desired position. The guidance for the patient could be done via a simple color-coding. For example, the colors green, yellow, blue and purple could be used to indicate the movement directions (e.g. up, down, left, and right) for reaching the desired position and/or orientation. A red light could be used to stop movement and indicate "best position". It should be noted, if the placement tracking arrangement 30 comprises the optical shape sensor, the fiber itself could be used as optical interface to visualize the color. In case of the body channel network the sensor itself has to be equipped with a multicolor LED as user interface. Furthermore, flashing lights might indicate a problem with the sensor position and/or orientation that might have moved or in case of skin contact sensor the interface condition (e.g., gel) might have changed.

It should be understood that the optical feedback might be used to indicate the need for action by the user. Further, the optical feedback may be done in a synchronized way with an examination arrangement, since no position and/or orientation change should be done during image acquisition but only before the next scan. Furthermore, audio guidance or guidance using tactile methods may support the action of the user. Thus, the user may be the patient itself. In other words, due to the guiding ability of the sensor positioning system 10, the user does not have to be a professional trained medical assistance. Furthermore, the measurement quality of the sensor arrangement 20 may be improved as the positioning of the at least one sensor are controlled and monitored. In addition, the placement of the sensor arrangement 20 may be accelerated as a direct feedback of an accurate placement is given to the user.

The sequence of the placement of the at least one sensor 14 may also be indicated by the optical interface, either by a unique color (white flashing indicating the sensor that has to be placed next) and/or temporal switching sequence. In case of display availability in the preparation area and/or inside the examination arrangement when the sensors should be placed the synchronized visualization of sensor number and senor position and/or orientation on the body may be displayed.
For example, the colors green, yellow, blue and purple could be used to indicate the movement directions (up, down, left, and right) for reaching the desired position and/or orientation, while a red light could be used to stop movement and indicate "best position". In another example, the output signal 45 of the processor 40 may instruct a robot system for autonomously positioning the at least one sensor 14 at the desired position. In this case, optical feedback and audio guidance are not required. Instead, the output signal 45 may signify a direction (e.g. left, right, etc.), distance (e.g. 5 cm, 15 cm, etc.), and/or orientation (e.g. clockwise rotation for 15°, 32°, etc.) for guiding the robot system to autonomously position the at least one sensor at the desired position.

Figs. 2A to 2C schematically show a placement tracking arrangement 30 according to some embodiments of the present disclosure. Fig. 2A schematically illustrates a single sensor 14 of the sensor arrangement 20 and a single placement tracker 32 of the placement tracking arrangement 30, which is removably mounted to the sensor 14.

In Figs. 2A and 2B, a sensor 14 and two placement trackers 32a, 32b are shown. In this example, the at least one reference point 12 is associated with a reference sensor, which, for simplicity, is also denoted with "12". However, it should be understood that more than two sensors and placement trackers might be used. The sensors of the sensor arrangement are configured to be placed on a patient's body to measure vital data of the patient.

As illustrated in Fig. 2A, the placement tracking arrangement 30 may be mounted to the reference sensor 12 and the sensor 14. An optical shape sensor 32c may be used to position the placement tracker 32b for the sensor 14 correctly relative to the placement tracker 32a for the reference sensor 12.

As illustrated in Fig. 2C, once the sensor 14 is correctly positioned relative to the reference sensor 12, the placement tracking arrangement 30 may be removed, thus leaving the sensor 14 at the correct position on the patient's body.

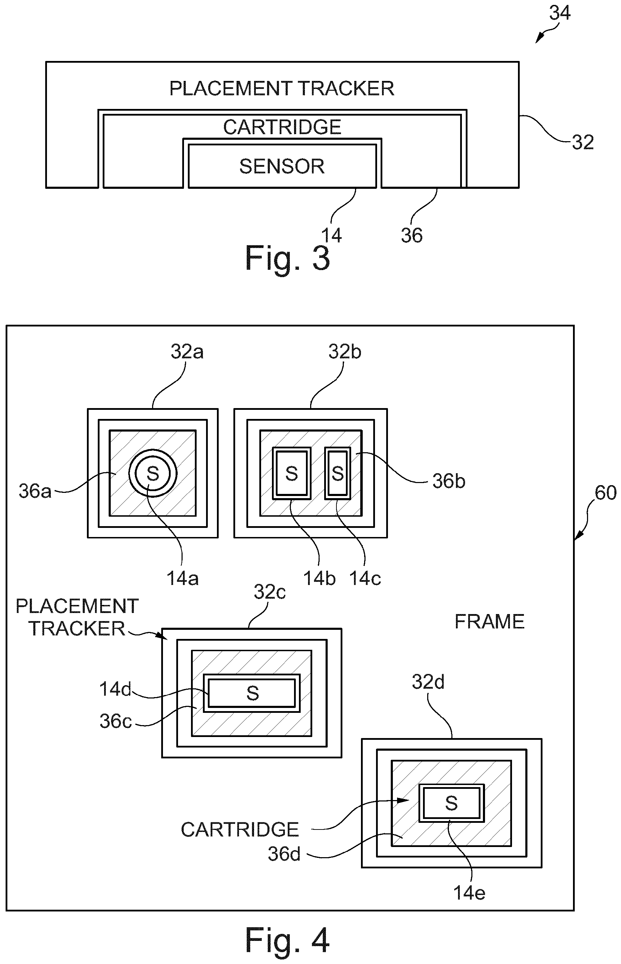

Fig. 3 schematically illustrates a placement tracking arrangement 30 according to some further embodiments of the present disclosure. In this example, the placement tracking arrangement 30 comprises one or more modular devices 34. Each modular device 34 has a respective placement tracker 32. Each modular device 34 is structured to accommodate at least one sensor 14.

In an example, one or more modular devices may be structured to accommodate a single sensor.

In an example, one or more modular devices may be structured to accommodate sensors of different sizes. This could be assisted by adding a cartridge feature to easily mount sensors of different sizes to a single placement tracking device format. An exemplary detachable cartridge 36 is illustrated in Fig. 3, which is removably mountable to the placement tracker 32. Thus, if different sensors need to be positioned on the body, a multiplicity of identical modular devices 34 with different detachable cartridges 36 may be used to accommodate different sensor formats.

In addition, whilst sensor positioning may only take a few minutes, the sensors may be worn by the patient for several hours or even days. Consequently, the hospital may require less placement tracker devices than sensor devices. Hence, it may be feasible to create placement trackers with higher specification or additional components to further increase their performance. For sensors, which are to be worn for many hours, they may be interconnected so that they can communicate among themselves and/or passively monitor their relative positions.

Whilst the modular approach allows for considerable flexibility, there may be workflow advantages if there is some standardization of the device set-up for more common procedures. Fig. 4 schematically illustrates a placement tracking arrangement 30 according to some further embodiments of the present disclosure. In this example, the placement tracker arrangement further comprises a frame device 60 that is removably mountable to the placement tracking arrangement 30. The frame device 60 is configured to accommodate each placement tracker of the placement tracking arrangement in a predefined position and/or predefined orientation that defines a default layout of the sensor arrangement on the patient's body.

In this example, as illustrated in Fig. 4, a default layout of four placement trackers 32a-d is defined by the frame device 60. The placement trackers 32a-d may be mounted, e.g. inserted, into the frame device 60. This frame device 60 thus ensures that the sensors 14, such as sensors 14a-e in Fig 4, are initially placed at approximately the correct relative positions and may assist in the speed of using the placement tracking feature to find the final position more efficiently.

Optionally, as illustrated in Fig. 4, each of the placement trackers 32a-d is removably mounted to a different detachable cartridge 36a-d to enable the mounting of different sensors. For example, as illustrated in Fig. 4, the detachable cartridge 36b is structured to accommodate and position two sensors 14b and 14c, which need to be positioned at a fixed distance from each other. The detachable cartridges 36a, 36c, and 36d are structured differently to accommodate a single sensor of different sizes and/or shapes.

Optionally, there may be a series of frame devices with different sizes. Prior to sensor positioning, one frame device may be chosen to match the size of the patient.

Optionally, for each frame size there may be a series of frame devices with different default layouts of the placement trackers. Each default layout is configured to be suitable for a particular procedure to be carried out.

Optionally, the frame device may be a flexible sheet with simple and less expensive sensors. These pre-configured flexible sheets with integrated sensors may be used as patient specific disposable, as the sensor setting might be connected to the patient's body for several hours and might not be reusable.

In use, the frame device 60 with placement trackers (e.g. placement trackers 32a-d in Fig. 4) and sensors (e.g. sensors 14a-e in Fig. 4), optionally with detachable cartridges (e.g. cartridges 36a-d) is positioned on the patient. Afterwards, the frame device 60 is removed, thus leaving the placement trackers (e.g. placement trackers 32a-d in Fig. 4) initially placed at approximately the correct relative positions. Each of the placement trackers (e.g. placement trackers 36a-d in Fig. 4) are then operated and guided around until at a desired position. Finally, the placement trackers (e.g. placement trackers 32a-d in Fig. 4) and the optional detachable cartridges (e.g. cartridges 36a-d in Fig. 4) are removed, leaving the sensors (e.g. sensors 14a-e in Fig. 4) at the desired position and/or orientation.

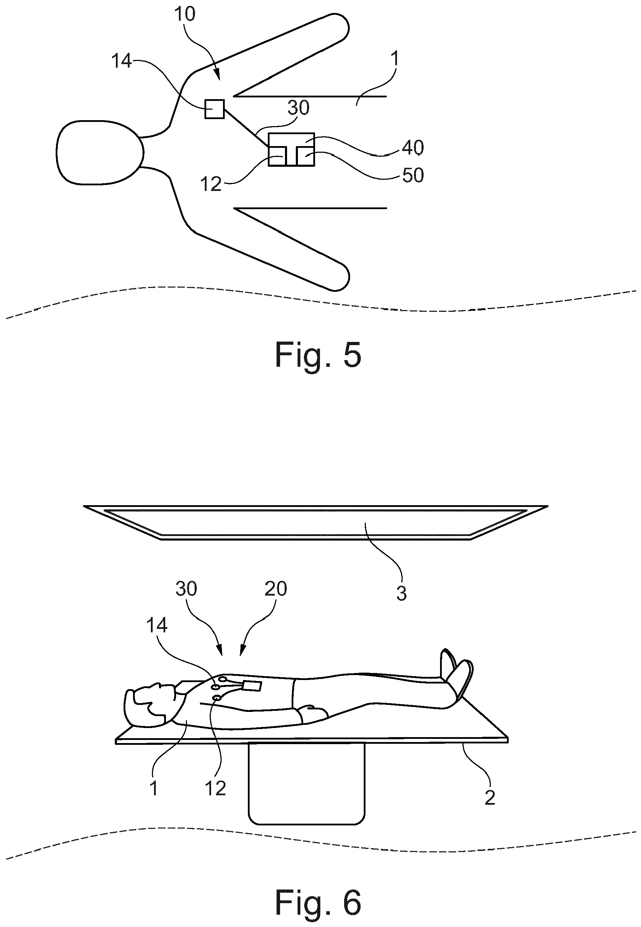

Fig. 5 shows a patient 1 with a sensor positioning system 10. In this example, the receiver 50 and the processor 40 are arranged in one common housing. A reference transmitter or a reference sensor may also be arranged in the common housing for positioning a reference point 12. Here, for simplicity, the reference transmitter or reference sensor is also denoted with "12". In this example, the reference position is the sternum of the patient 1. Further, the placement tracking arrangement 30 is used to track and provide a feedback about a position and/or orientation of the sensor 14 relative to the reference point 12 on the patient's body. The desired position and/or orientation for the sensor 14 is received by the receiver 50. Subsequently the sensor 14 is moved over the patient's body meanwhile the actual position and/or orientation of the sensor 14 is tracked by the placement tracking arrangement 30. The processor 40 is configured to compare the actual position and/or orientation with the desired position and/or orientation of the sensor 14. Further, the processor 40 generates the output signal in form of an optical and/or acoustical feedback, e.g. illuminating the at least one sensor, to guide the user to place the sensor at the desired position. Alternatively, the output signal may instruct a robot system to autonomously position the at least one sensor at the desired position and/or orientation.

In Fig. 6, an examining table 2, on which a patient 1 lies, is shown. The patient 1 is lying down e.g. for an MRI or CT scan. A ceiling mounted mirror 3 may be provided that allows the patient 1 to see himself and the sensor arrangement 20 from above. If the left, top, right and bottom edges of the mirror may a built-in lighting element then those edges can be used to intuitively indicate in which direction the sensor 14 has to be moved. For example if the right hand edge lights up green it means that the user needs to move the sensor 14 in the direction of that edge. If it lights up orange it means that the user is almost there, if it lights up red the user needs to stop. If the user overshoots and needs to reverse motion then the left hand edge lights up.

In another example (not shown), the processor 40 may generate the output signal for guiding a robot system to autonomously place the sensor 14 at the desired position.

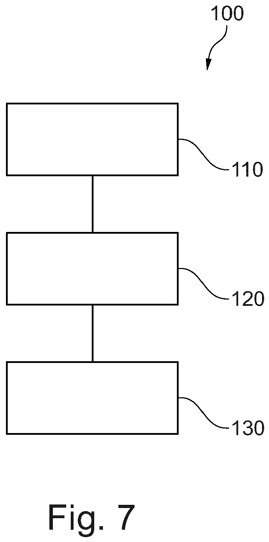

Fig. 7 shows a flowchart of a method 100 for positioning and placement of a sensor arrangement on a patient's body according to some embodiments of the present disclosure.

In step 110, a sensor arrangement is mounted to a placement tracking arrangement. The sensor arrangement comprises at least one sensor. The placement tracking arrangement comprises one or more placement trackers. Each placement tracker is removably mountable to one or more sensors. Examples of the placement trackers may include, but are not limited to, an optical shape sensor and a RF transceiver device operating at a body channel communication frequency.

Optionally, the placement tracking arrangement may comprise one or more modular devices. Each modular device may have a respective placement tracker, which may be removably attached to a detachable cartridge. The detachable cartridge may be structured to accommodate one or more sensors, which are removably mountable to the detachable cartridge. The placement tracker in each modular device may have a universal size. An exemplary modular device is illustrated in Fig. 3.

Optionally, a frame device may be provided that is removably mountable to the placement tracking arrangement. The frame device is configured to accommodate each placement tracker of the placement tracking arrangement in a predefined position and/or predefined orientation that defines a default layout of the sensor arrangement on the patient's body. Some placement trackers may comprise a detachable cartridge for accommodating one or more sensors. The frame device may comprise a standard or flexible interconnect for the sensor arrangement. The frame device may be a flexible sheet with integrated sensor arrangement. An exemplary frame device is illustrated in Fig. 4.

In step 120, the placement tracking arrangement tracks and provides a feedback about a position and/or orientation of the at least one sensor relative to at least one reference point on the patient's body by the placement tracking arrangement to determine an actual position and/or orientation of the at least one sensor on the patient's body.

The position of at least one reference point may be received by a receiver. A reference transmitter or a reference sensor may be used for positioning at the at least one reference point, which may form the basis for the positioning of the at least one sensor of the sensor arrangement.

In step 130, a processor compares the actual position and/or orientation with the desired position and/or orientation of the at least one sensor to generate an output signal indicating a difference between the actual position and/or orientation and the desired position and/or orientation of the at least one sensor.

The receiver may be configured to receive the desired position and/or orientation of the at least one sensor.

Optionally, it may be checked whether the at least one sensor is at the desired position. If yes, a feedback is issued signifying that the at least one sensor at the desired position. If no, the output signal may guide a user (e.g. a patient) via the optical feedback or guide a robot system via instructions to place the at least one senor at the desired position.

Optionally, once the sensor arrangement is positioned at the desired position and/or orientation, the placement tracking arrangement may be detached from the sensor arrangement.

It should also be understood that, unless clearly indicated to the contrary, in any methods claimed herein that include more than one step or act, the order of the steps or acts of the method is not necessarily limited to the order in which the steps or acts of the method are recited.

All definitions, as defined and used herein, should be understood to control over dictionary definitions, definitions in documents incorporated by reference, and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of' or "exactly one of," or, when used in the claims, "consisting of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of."

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of' and "consisting essentially of' shall be closed or semi-closed transitional phrases, respectively.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both, a computer program that right from the beginning uses the invention and a computer program that by means of an up-date turns an existing program into a program that uses the invention.

Further on, the computer program element might be able to provide all necessary steps to fulfil the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section.

A computer program may be stored and/or distributed on a suitable medium, such as an optical storage medium or a solid state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

While several inventive embodiments have been described and illustrated herein, those of ordinary skill in the art will readily envision a variety of other means and/or structures for performing the function and/or obtaining the results and/or one or more of the advantages described herein, and each of such variations and/or modifications is deemed to be within the scope of the inventive embodiments described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the inventive teachings is/are used. Those skilled in the art will recognize, or be able to ascertain using no more than routine experimentation, many equivalents to the specific inventive embodiments described herein. It is, therefore, to be understood that the foregoing embodiments are presented by way of example only and that, within the scope of the appended claims and equivalents thereto, inventive embodiments may be practiced otherwise than as specifically described and claimed. Inventive embodiments of the present disclosure are directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the inventive scope of the present disclosure.

## Claims

1. A sensor positioning system (10) for positioning and placement of a sensor arrangement on a patient's body, comprising:
- a sensor arrangement (20) comprising at least one sensor (14, 14a, 14b, 14c, 14d, 14e);
- a placement tracking arrangement (30) comprising one or more placement trackers (32, 32a, 32b, 32c, 32d), each placement tracker being removably mountable to one or more sensors; and
- a processor (40);
wherein the placement tracker arrangment is configured to track and provide a feedback about a position and/or orientation of the at least one sensor relative to at least one reference point on the patient's body to determine an actual position and/or orientation of the at least one sensor on the patient's body; and
wherein the processor is configured to compare the actual position and/or orientation of the at least one sensor with a desired position and/or orientation of the at least one sensor and to generate an output signal indicating a difference between the actual position and/or orientation and the desired position and/or orientation of the at least one sensor.

2. The sensor positioning system according to claim 1,
wherein the placement tracking arrangement comprises one or more modular devices (34), each modular device having a respective placement tracker; and
wherein each modular device is structured to accommodate at least one sensor.

3. The sensor positioning system according to claim 2,
wherein each modular device comprises a detachable cartridge (36, 36a, 36b, 36c, 36d) that is removably mountable to the placement tracker of the respective modular device; and
wherein the detachable cartridge is structured to accommodate one or more sensors, which are removably mountable to the detachable cartridge.

4. The sensor positioning system according to claim 2 to 3,
wherein the placement tracker in each modular device has a universal size.

5. The sensor positioning system according to any one of the preceding claims, further comprising:
- a frame device (60) removably mountable to the placement tracking arrangement;
wherein the frame device is configured to accommodate each placement tracker of the placement tracking arrangement in a predefined position and/or predefined orientation that defines a default layout of the sensor arrangement on the patient's body.

6. The sensor positioning system according to claim 5,
wherein at least one placement tracker on the frame device comprises a detachable cartridge for accommodating one or more sensors.

7. The sensor positioning system according to claim 5 or 6,
wherein the frame device comprises a standard or flexible interconnect for the sensor arrangement.

8. The sensor positioning system according to any one of claims 5 to 7,
wherein the frame device is a flexible sheet; and
wherein the sensor arrangement is an integrated part of the flexible sheet.

9. The sensor positioning system according to any one of the preceding claims,
wherein the at least one reference point comprises an anatomical marker of the patient's body.

10. The sensor positioning system according to any one of the preceding claims,
wherein the one or more placement trackers comprise at least one of an optical shape sensor and a radio-frequency, RF, transceiver device operating at a body channel communication frequency.

11. The sensor positioning system according to any one of the preceding claims,
wherein the processor is configured to provide a guidance for the placement of the at least one sensor at the desired position and/or orientation based on the comparison of the actual position and/or orientation with the desired position and/or orientation of the at least one sensor.

12. An examination arrangement, comprising:
- an examination apparatus for examination of an object of interest; and
- a sensor positioning system according to any one of the preceding claims.

13. A method (100) for positioning and placement of a sensor arrangement on a patient's body, comprising the steps of:
- mounting (110) a sensor arrangement to a placement tracking arrangement, wherein the sensor arrangement comprises at least one sensor, and wherein the placement tracking arrangement comprises one or more placement trackers, each placement tracker being removably mountable to one or more sensors;
- tracking (120), by the placemenent tracking arrangement, and providing a feedback about a position and/or orientation of the at least one sensor relative to at least one reference point on the patient's body by the placement tracking arrangement to determine an actual position and/or orientation of the at least one sensor on the patient's body;
- comparing (130), by a processor, the actual position and/or orientation with the desired position and/or orientation of the at least one sensor to generate an output signal indicating a difference between the actual position and/or orientation and the desired position and/or orientation of the at least one sensor.

14. A computer program element for controlling a sensor positioning system according to any one of claims 1 to 11 or an examination arrangement of claim 12, which when being executed by a processor is configured to carry out the method according to claim 13.

15. A computer readable medium having stored thereon the program element of claim 14.
